# EUROPÄISCHE PATENTANMELDUNG

(11) **EP 2 926 801 A1**
(43) Veröffentlichungstag der Anmeldung: **07.10.2015**
(21) Anmeldenummer: 15160688.6
(22) Anmeldetag: 25.03.2015
(51) Int. Cl.: A61K 8/39, A61K 8/44, A61K 8/49, A61Q 19/08, A61K 8/06

(54) **KOSMETISCHE WASSER-IN-ÖL-EMULSION**

(30) Priorität: 02.04.2014 DE 102014206332
(71) Anmelder: Beiersdorf AG, 20253 Hamburg (DE)
(72) Erfinder: Neumann, Yvonne, 22529 Hamburg (DE); Lüttig, Kaja, 20253 Hamburg (DE)

(57) **Zusammenfassung**

Es werden eine Wasser-in-Öl-Emulsion zum Einkapseln von hydrophilen Wirkstoffen und ein Verfahren zur Herstellung der Emulsion beschrieben.

## Beschreibung

### Gebiet der Erfindung

Die vorliegende Erfindung betrifft eine Wasser-in-Öl-Emulsion zum Einkapseln von hydrophilen Wirkstoffen, insbesondere eine Wasser-in-Öl-Emulsion mit einer Kombination von Emulgatoren und einer Viskosität im Bereich von 1000 bis 4000 mPa.s.

### Allgemeiner Stand der Technik

Der Wunsch, schön und attraktiv auszusehen, ist im Menschen auf natürliche Weise verwurzelt. Obwohl sich das Schönheitsideal im Verlauf der Zeit verändert hat, war der Wunsch nach makellosem äußerlichem Aussehen immer das Ziel des Menschen. Zustand und Erscheinungsbild der Haut sind ein wesentlicher Teil eines schönen und attraktiven äußerlichen Erscheinungsbilds.

Damit die Haut ihre biologischen Funktionen vollständig erfüllen kann, muss sie regelmäßig gereinigt und gepflegt werden. Das Reinigen der Haut dient also der Entfernung von Schmutz, Schweiß und Resten von toten Hautteilchen, die eine ideale Brutstätte für Pathogene und Parasiten aller Art bilden. Hautpflegeprodukte dienen in erster Linie dazu, der Haut Feuchtigkeit und Fett zuzuführen. Häufig werden ihnen Wirkstoffe hinzugefügt, die die Haut regenerieren und zum Beispiel dazu dienen sollen, ihre vorzeitige Alterung (d.h. das Auftreten von Fältchen und Falten) zu verhindern und zu reduzieren.

Hautpflegeprodukte bestehen üblicherweise aus Emulsionen. Unter Emulsionen versteht man im Allgemeinen heterogene Systeme, die aus zwei Flüssigkeiten, die unmischbar oder nur begrenzt mischbar sind, bestehen und die üblicherweise als Phasen bezeichnet werden, und in denen eine der beiden Flüssigkeiten in Form von sehr feinen Tröpfchen in der anderen Flüssigkeit dispergiert ist. Äußerlich und mit bloßem Auge betrachtet, erscheinen Emulsionen homogen.

Wenn die beiden Flüssigkeiten Wasser und Öl sind und Öltröpfchen in Wasser in feindispergierter Form vorliegen, so handelt es sich um eine Öl-in-Wasser-Emulsion (O/W-Emulsion, z.B. Milch). Der Grundcharakter einer O/W-Emulsion ist durch das Wasser definiert. Im Falle einer Wasser-in-Öl-Emulsion (W/O-Emulsion, z.B. Butter) ist das Prinzip umgekehrt, und der Grundcharakter wird hier von dem Öl bestimmt. Bei der Herstellung einer Emulsion wird die wässrige Phase unter Rühren mit der Lipidphase (Ölphase) kombiniert.

Um Emulsionen längere Zeit stabil zu halten und die Trennung der Phasen zu verhindern, werden die Emulsionen mit sogenannten Emulgatoren versetzt. Im Allgemeinen sind Emulgatoren Moleküle mit einem polaren hydrophilen Strukturelement und einem unpolaren lipophilen Strukturelement. In den späten 40iger Jahren wurde ein System entwickelt, das dahingehend ausgelegt war, um die Auswahl von Emulgatoren zu erleichtern. Jedem Emulgator wurde ein sogenannter HLB-Wert (eine dimensionslose Zahl zwischen 0 und 20) zugeordnet, die angibt, ob eine bevorzugte Wasserlöslichkeit oder Öllöslichkeit vorliegt. Zahlen unter 9 kennzeichnen öllösliche, hydrophobe Emulgatoren, und Zahlen über 10 solche, die wasserlöslich, hydrophil sind.

Bei der Herstellung von W/O-Emulsionen mit hydrophilen Wirkstoffen, zum Beispiel hydrophilen Wirkstoffen zur Vorbeugung oder Reduktion der vorzeitigen Alterung, muss man eine W/O-Emulsion bereitstellen, die ausreichend stabil ist, um eine pharmakologisch wirksame Menge des hydrophilen Wirkstoffs in der W/O-Emulsion zu halten.

Es wäre vorteilhaft, über eine W/O-Emulsion zum Einkapseln von hydrophilen Wirkstoffen und über ein Verfahren zur Herstellung solch einer W/O-Emulsion, die während der Herstellung und/oder Lagerung stabil ist, zu verfügen.

In der DE 10357640 A1 werden kapselförmige W/O-Emulsionen beschrieben, die sich aufgrund einer bestimmten Abmischung von Wachsen, Lipiden, Emulgatoren, natürlichen und/oder synthetischen Polymere und/oder Mischungen daraus ergeben.

Als bevorzugte Emulgatoren werden dem Fachmann geläufige W/O-Emulgatoren genannt wie u.a. Poylethylenglycol-45/Dodecylglycolcopolymer, Polyglyceryl-3-Diisostearat, PEG-30 Dipolyhydroxystearat, Sorbitanisostearat, Sorbitanstearat, Glyceryl Isostearat, Glyceryl Stearat und Sorbitan Oleat.

Die DE 202006001274 U1 beschreibt kosmetische Zubereitungen umfassend ein oder mehrere flüchtige Lipide und ein oder mehrere Parfümöle. Die Zubereitung kann sowohl als O/W- als auch W/O-Emulsion formuliert werden. Als bevorzugte W/O-Emulgatoren werden PEG-30 Dipolyhydroxystearat, Polyglyceryl-3-Diisostearat, PEG-40 Sorbitan Perisostearat genannt.

Die DE 202006005494 U1 beschreibt W/O Emulsionen als Selbstbräunungszubereitungen umfassend u.a. Polyglyceryl-3-Diisostearat und PEG-40 Sorbitanisostearat sowie DHA als Bräunungsmittel.

Gemäß einem ersten Aspekt der Erfindung wird eine Wasser-in-Öl-Emulsion zum Einkapseln eines hydrophilen Wirkstoffs bereitgestellt, wobei die Wasser-in-Öl-Emulsion ein Emulgatorsystem umfasst, das Sorbitanstearat oder ein Derivat davon und Polyglycerylstearat oder ein Derivat davon umfasst.

Die Erfinder der vorliegenden Erfindung haben unerwarteterweise gefunden, dass eine Wasser-in-Öl-Emulsion, die ein Emulgatorsystem umfasst, das zumindest die Emulgatoren Sorbitanstearat und Polyglycerylstearat umfasst, fähig ist, einen hydrophilen Wirkstoff in einer Menge, die zuvor nicht möglich war, zu stabilisieren und einzukapseln. Solche Emulsionen können als Formulierungen für die topische Verabreichung von Wirkstoffen verwendet werden, um zum Beispiel der vorzeitigen Alterung vorzubeugen oder diese zu reduzieren.

Die Verwendung von Sorbitanstearat und Polyglycerylstearat als kombinierte Emulgatoren in einer Wasser-in-Öl-Emulsion führt zu einer Emulsion mit niedriger Viskosität im Bereich von 1000 bis 4000 mPa.s. Ohne an eine Theorie gebunden sein zu wollen, ist man der Meinung, dass es genau diese Emulgatorkombination ist, die zu dieser Viskosität führt, die ein stabiles Einkapseln von hydrophilen Wirkstoffen in Mengen von bis zu 5 Gew.-% ermöglicht. In einer Ausführungsform der vorliegenden Erfindung sind daher Sorbitanstearat und Polyglycerylstearat in der Wasser-in-ÖI-Emulsion in solch einer Menge vorhanden, dass die Wasser-in-Öl-Emulsion eine Viskosität im Bereich von 1000 bis 4000 mPa.s aufweist. In einer alternativen Ausführungsform sind Sorbitanstearat und Polyglycerylstearat in der Wasser-in-ÖI-Emulsion in solch einer Menge vorhanden, dass die Wasser-in-Öl-Emulsion eine Viskosität im Bereich von 2000 bis 4000 mPa.s aufweist. In einer alternativen Ausführungsform sind Sorbitanstearat und Polyglycerylstearat in der Wasser-in-ÖI-Emulsion in solch einer Menge vorhanden, dass die Wasser-in-Öl-Emulsion eine Viskosität im Bereich von 3000 bis 4000 mPa.s aufweist.

Gemäß einem zweiten Aspekt der vorliegenden Erfindung wird ein Verfahren zur Herstellung der Wasser-in-Öl-Emulsion des ersten Aspekts bereitgestellt, wobei man bei dem Verfahren Sorbitanstearat oder ein Derivat davon und Polyglycerylstearat oder ein Derivat davon mit einer Ölphase mischt und die Ölphase erhitzt, und die erhitzte Ölphase mit einer erhitzten wässrigen Phase unter Bildung der Wasser-in-Öl-Emulsion homogenisiert.

Die Wasser-in-ÖI-Emulsion der vorliegenden Erfindung umfasst Sorbitanstearat oder ein Derivat davon als Teil des Emulgatorsystems. Sorbitan ist eine Mischung aus isomeren organischen Verbindungen, die durch Entwässern von Sorbit entstehen. Die Mischung kann schwanken, besteht jedoch üblicherweise aus 1,4-Anhydrosorbitol, 1,5-Anhydrosorbitol und 1,4,3,6-Dianhydrosorbitol.

Falls nicht anders erwähnt, ist der Begriff "Sorbitanstearat" dahingehend zu interpretieren, dass er Sorbitan beinhaltet, das mit einer oder mehreren Stearatgruppen, mit einem oder mehreren unterschiedlichen Substitutionsmustern der Stearatgruppe und/oder unterschiedlichen Stearatregioisomeren verestert worden ist. So zum Beispiel umfasst der Begriff "Sorbitanstearat" innerhalb seines Umfangs Verbindungen wie Sorbitanmonostearat, Sorbitandistearat, Sorbitantristearat, Sorbitanisostearat, Sorbitandiisostearat und Sorbitanperisostearat. In einer Ausführungsform der Erfindung umfasst das Sorbitanstearat daher eines oder mehrere aus der Reihe Sorbitanmonostearat, Sorbitandistearat, Sorbitantristearat, Sorbitanisostearat, Sorbitandiisostearat und Sorbitanperisostearat.

Weiterhin umfasst der Begriff "Sorbitanstearat" innerhalb seines Umfangs Sorbitanstearatderivate, einschließlich Sorbitanstearatverbindungen wie die oben erwähnten, die mit Polyetherverbindungen konjugiert sind. So zum Beispiel umfasst der Begriff "Sorbitanstearat" Verbindungen wie diejenigen, die oben aufgezählt sind, die mit einem Polyethylenglykolrest, d.h. einer PEG-Gruppe, konjugiert sind.

In einer Ausführungsform umfasst das Sorbitanstearat Sorbitanperisostearat, das mit einer PEG-Gruppe konjugiert ist. Die PEG-Gruppe kann eines oder mehrere aus der Reihe PEG-2, PEG-3, PEG-4, PEG-5, PEG-6, PEG-7, PEG-8, PEG-9, PEG-10, PEG-12, PEG-14, PEG-15, PEG-20, PEG-25, PEG-30, PEG-40 oder PEG-50 umfassen. Falls nicht anders erwähnt, geben die Zahlen die Anzahl der Ethylenoxidmonomereinheiten in dem Rest an. So zum Beispiel enthält ein PEG-9 9 Ethylenoxid-Monomereinheiten..

In einer Ausführungsform umfasst die erfindungsgemäße Wasser-in-Öl-Emulsion bis zu 3 Gew.-% Sorbitanstearat oder eines oder mehrere Derivate davon, in Bezug auf das Gesamtgewicht der Emulsion. In einer Ausführungsform umfasst die erfindungsgemäße Wasser-in-Öl-Emulsion weniger als 3 Gew.-%, weniger als 2,9 Gew.-%, weniger als 2,8 Gew.-%, weniger als 2,7 Gew.-%, weniger als 2,6 Gew.-%, weniger als 2,5% Sorbitanstearat oder eines oder mehrere Derivate davon, in Bezug auf das Gesamtgewicht der Emulsion.

Alternativ dazu umfasst die erfindungsgemäße Wasser-in-Öl-Emulsion mindestens 0,5 Gew.-%, mindestens 0,6 Gew.-%, mindestens 0,7 Gew.-%, mindestens 0,8 Gew.-%, mindestens 0,9 Gew.-%, mindestens 1,0 Gew.-% Sorbitanstearat oder ein oder mehrere Derivate davon, in Bezug auf das Gesamtgewicht der Emulsion.

Die erfindungsgemäße Wasser-in-Öl-Emulsion umfasst auch Polyglycerylstearat als Teil des Emulgatorsystems. Falls nicht anders erwähnt, ist der Begriff "Polyglycerylstearat" dahingehend zu verstehen, dass er Polyglycerylreste mit mindestens zwei Glyceroleinheiten, mit einem oder mehreren unterschiedlichen Substitutionsmustern der Stearatgruppe beinhaltet. So zum Beispiel umfasst der Begriff "Polyglyceryl" innerhalb seines Umfangs Verbindungen wie Polyglyceryl-2, Polyglyceryl-3, Polyglyceryl-4, Polyglyceryl-5, Polyglyceryl-6, Polyglyceryl-7, Polyglyceryl-8, Polyglyceryl-9 oder Polyglyceryl-10. Die Zahlen sind dahingehend zu verstehen, dass sie die Anzahl der Glyceryleinheiten in dem Polyglycerylrest bedeuten; anders ausgedrückt enthält ein Polyglyceryl-3-Rest drei Glyceryleinheiten.

Der Begriff "Polyglycerylstearat" ist dahingehend zu verstehen, dass er Polyglycerylreste beinhaltet, die mit einer oder mehreren Stearatgruppen, mit einem oder mehreren unterschiedlichen Substitutionsmustern der Stearatgruppe und/oder unterschiedlichen Stearatregioisomeren verestert worden sind. So zum Beispiel umfasst der Begriff "Polyglycerylstearat" innerhalb seines Umfangs Verbindungen mit einem Veresterungsmuster, das eines oder mehrere aus der Reihe Monostearat, Distearat, Tristearat, Isostearat, Diisostearat und Perisostearat beinhaltet. In einer Ausführungsform der Erfindung umfasst das Polyglycerylstearat daher eines oder mehrere aus der Reihe Polyglycerylmonostearat, Polyglyceryldistearat, Polyglyceryltristearat, Polyglycerylisostearat, Polyglyceryldiisostearat und Polyglycerylperisostearat.

In einer Ausführungsform umfasst die erfindungsgemäße Wasser-in-Öl-Emulsion bis zu 2 Gew.-% Polyglycerylstearat oder eines oder mehrere Derivate davon, in Bezug auf das Gesamtgewicht der Emulsion. In einer Ausführungsform umfasst die erfindungsgemäße Wasser-in-Öl-Emulsion weniger als 2 Gew.-%, weniger als 1,9 Gew.-%, weniger als 1,8 Gew.-%, weniger als 1,7 Gew.-%, weniger als 1,6 Gew.-%, weniger als 1,5% Polyglycerylstearat oder eines oder mehrere Derivate davon, in Bezug auf das Gesamtgewicht der Emulsion.

Alternativ dazu umfasst die erfindungsgemäße Wasser-in-Öl-Emulsion mindestens 0,5 Gew.-%, mindestens 0,6 Gew.-%, mindestens 0,7 Gew.-%, mindestens 0,8 Gew.-%, mindestens 0,9 Gew.-%, mindestens 1,0 Gew.-% Polyglycerylstearat oder ein oder mehrere Derivate davon, in Bezug auf das Gesamtgewicht der Emulsion.

In einer erfindungsgemäßen Ausführungsform umfasst die Wasser-in-Öl-Emulsion 1,5 bis 2,0 Gew.-% Sorbitanstearat oder ein Derivat davon und 1,0 bis 1,5 Gew.-% Polyglycerylstearat oder ein Derivat davon. Eine Wasser-in-Öl-Emulsion gemäß dieser Ausführungsform weist eine Viskosität im Bereich von 3000 bis 4000 mPa.s auf und eignet sich daher besonders für das Einkapseln von hydrophilen Wirkstoffen in einer Menge von bis zu 8 Gew.-%.

Erfindungsgemäß bevorzugt besteht das Emulgatorsystem nur aus einem Sorbitanstearat und einem Polyglycerylstearat, insbesondere Polyglyceryl-3 Diisostearat und PEG-40 Sorbitan Perisostearat.

Bei dem Wirkstoff, den es einzukapseln gilt, kann es sich um einen beliebigen kosmetischen hydrophilen Wirkstoff handeln, den es in eine Formulierung für die topische Verabreichung einzukapseln gilt. So zum Beispiel kann es sich bei dem Wirkstoff um einen Wirkstoff gegen das Altern wie Carnitin handeln.

Alternativ können neben mindestens einem hydrophilen Wirkstoff weitere Wirkstoffe enthalten sein, wie beispielsweise Coenzym Q10 oder Vitamine, z.B. Vitamin C.

Besonders bevorzugt ist es, dass kosmetische hautpflegende Wirkstoffe in der Formulierung enthalten sind.

Folgende Wirkstoffe können vorteilhaft enthalten sein, gewählt aus der Gruppe der Verbindungen Folsäure, Coenzym Q10, alpha-Glucosylrutin, Carnitin, Carnosin, Coffein, natürliche und/oder synthetische Isoflavonoide, Glycerylglucose, Kreatin, Kreatinin, Taurin, Magnolia, ß-Alanin und/oder Licochalcon A.

Dihydroxyaceton ist explizit als Wirkstoff ausgeschlossen.

Der hydrophile Wirkstoff, den es einzukapseln gilt, kann in einer Menge vorliegen, bei der es sich um eine bei Vorliegen in einer Formulierung für die topische Verabreichung kosmetisch, dermatologisch oder pharmakologisch wirksame Menge handelt. So zum Beispiel kann der Wirkstoff in einer Menge von bis zu 8 Gew.-% vorliegen. Alternativ dazu kann der Wirkstoff in einer Menge von 8 Gew.-% oder weniger, zum Beispiel 7 Gew.-% oder weniger, 6 Gew.-% oder weniger, 5 Gew.-% oder weniger, 4 Gew.-% oder weniger, 3 Gew.-% oder weniger oder 2 Gew.-% oder weniger, vorliegen.

Alternativ dazu kann der Wirkstoff in einer Menge von 1 Gew.-% oder mehr, zum Beispiel 2 Gew.-% oder mehr, 3 Gew.-% oder mehr, 4 Gew.-% oder mehr, 5 Gew.-% oder mehr, 6 Gew.-% oder mehr oder 7 Gew.-% oder mehr, vorliegen.

Die erfindungsgemäße Wasser-in-Öl-Emulsion kann auch einen oder mehrere Zusatzstoffe des Typs, der routinemäßig in Kosmetikpräparaten verwendet wird, wie Antioxidantien, Konservierungsmittel, Stabilisatoren, Emmolientien, pH-Regulatoren und Duftstoffe, umfassen.

So zum Beispiel kann die Wasser-in-Öl-Emulsion weiterhin ein oder mehrere Antioxidantien umfassen. Solche Antioxidantien sind gut fachbekannt; dazu können eines oder mehrere aus der Reihe Tocopherol, Tocopherolacetat oder 2,6-Di-tert.-butyl-4-methylphenol zählen.

Gegebenenfalls kann die Wasser-in-Öl-Emulsion weiterhin ein oder mehrere Konservierungsmittel, insbesondere ein oder mehrere Parabene und/oder Phenoxyethanol und/oder Kaliumsorbat, umfassen.

Gegebenenfalls kann die Wasser-in-Öl-Emulsion weiterhin ein oder mehrere Stabilisatoren, zum Beispiel polymere Stabilisatoren, wie sie fachbekannt sind, umfassen. Zu geeigneten Beispielen zählen Acrylatpolymere, Carbomere, Stärken und Xanthangummi. Zu alternativen Stabilisatoren zählen anorganische Stabilisatoren wie Magnesiumsulfat.

Gegebenenfalls kann die Wasser-in-Öl-Emulsion weiterhin ein oder mehrere Emolientien des Typs, der routinemäßig in derartigen Präparaten eingesetzt wird, umfassen. Zu geeigneten Emolientien zählen Alkylpalmitatverbindungen wie Isopropylpalmitat sowie Paraffinum liquidum und Isoparaffine.

Gegebenenfalls kann die Wasser-in-Öl-Emulsion weiterhin ein oder mehrere

Neutralisierungsmittel oder pH-Regulatoren, wie sie fachbekannt sind, umfassen. Zu geeigneten Mitteln zählen Puffersysteme wie Citronensäure und Natriumcitrat.

Gegebenenfalls kann die Wasser-in-Öl-Emulsion weiterhin eine oder mehrere kosmetische Duftstoffsubstanzen wie Limonen, Linalool, Benzylbenzoat, Hydroxyisohexyl-3-cyclohexen, Carboxaldehyd, Hexylcinnamal, Benzylsalicylat, Eugenol, Butylphenylmethylpropional, alpha-Isomethylionon, Citronellol, Cumarin, Geraniol, Cinnamylalkohol oder Citral umfassen.

Man weiß, dass die Wasserphase der Wasser-in-Öl-Emulsion Wasser als Lösungsmittel umfasst, gegebenenfalls gemeinsam mit anderen Komponenten oder Hilfslösungsmitteln, die wasserlöslich sind, wie Alkoholen, und dass die Ölphase in der Wasser-in-Öl-Emulsion jegliche lipophile Komponente, wie ein lipophiles Emolliens, zum Beispiel Paraffinum liquidum, umfassen kann.

Die erfindungsgemäße Wasser-in-Öl-Emulsion kann dadurch hergestellt werden, dass man die Ölphase und die Wasserphase unabhängig voneinander erhitzt und dann die Ölphase und die Wasserphase vermischt.

In einer Ausführungsform der Erfindung werden das Sorbitanstearat und Polyglycerylstearat zu der Ölphase gegeben, bevor diese zu der wässrigen Phase gegeben wird.

Die wässrige Phase kann auf eine Temperatur von 50 bis 95°C, zum Beispiel eine Temperatur von 75 bis 90°C oder eine Temperatur von ungefähr 75°C oder 85°C, erhitzt werden. Der Begriff "ungefähr" soll in diesem Zusammenhang nur die leichten Temperaturvariationen, die für solche Verfahren typisch sind, kennzeichnen.

Die Ölphase kann auf eine Temperatur von 50 bis 95°C, zum Beispiel eine Temperatur von 75 bis 90°C oder eine Temperatur von ungefähr 75°C oder 85°C, erhitzt werden. Der Begriff "ungefähr" soll in diesem Zusammenhang nur die leichten Temperaturvariationen, die für solche Verfahren typisch sind, kennzeichnen.

Als Homogenisatoren verwendet man erfindungsgemäß vorzugsweise Mischer von Becomix oder Krieger, in die die erhitzte wässrige Phase und die erhitzte Ölphase gegeben und unter Bildung der Wasser-in-Öl-Emulsion homogenisiert werden.

Das Homogenisieren kann über einen Zeitraum von 2 bis 25 Minuten erfolgen.

Die Rührgeschwindigkeit während des Homogenisierens kann 500 bis 2000 U/min sein, zum Beispiel eine Rührgeschwindigkeit von 1000 bis 1400 U/min oder eine Rührgeschwindigkeit von 1200 U/min (± 50 U/min).

Die während des Homogenisierens gebildete Wasser-in-Öl-Emulsion kann auf eine Temperatur von 25 bis 40°C, zum Beispiel eine Temperatur von ungefähr 35°C, gekühlt werden. Der Begriff "ungefähr" soll in diesem Zusammenhang nur die leichten Temperaturvariationen, die für solche Verfahren typisch sind, kennzeichnen.

Jegliche Zusatzstoffe, die in der Emulsion vorliegen, wie Antioxidantien, Konservierungsmittel, Emollientien oder pH-Regulatoren, können miteinander vermischt werden, bevor sie zu der Emulsion gegeben werden.

Erstaunlicherweise eignet sich die erfindungsgemäße Emulsion aufgrund des Emulgatorsystems zur in-situ Verkapselung hydrophiler Wirkstoffe mit der Polyurethan/Core-Shell Technik.

PU Core-Shell Emulsion Polymer Partikel sind im Stand der Technik bekannt, wie beispielsweise in US 4,644,303 oder US 5,137,961 beschrieben.

In einer Ausführungsform können die Zusatzstoffe, falls vorhanden, in Ethanol gelöst werden, bevor sie zu der Emulsion gegeben werden. Das Hinzugeben von beliebigen Zusatzstoffen kann bei einer Temperatur von unter 30°C erfolgen.

### Beispiel

Das folgende Beispiel ist dahingehend zu verstehen, dass es lediglich als Beispiel oder Veranschaulichung der Anwendung der Prinzipien der vorliegenden Erfindung gilt. Der Fachmann kann sich zahlreiche Modifikationen und alternative Zusammensetzungen und Verfahren ausdenken, ohne vom Erfindungsgedanken und -umfang der vorliegenden Erfindung abzuweichen. Diese Beispiele sind daher nicht als Einschränkungen der vorliegenden Offenbarung anzusehen, aber dienen lediglich der Lehre der Herstelllung von Zusammensetzungen der vorliegenden Offenbarung.

Falls nicht anders erwähnt, folgt die gesamte Nomenklatur der chemischen Verbindungen in der obigen Beschreibung der Erfindung und in dem Beispiel unten dem INCI-Nomenklatursystem (International Nomenclature of Cosmetic Ingredients), mit dem der Fachmann vertraut ist.

### Beispiel 1

Gemäß der oben beschriebenen allgemeinen Vorschrift wurde eine Wasser-in-ÖI-Emulsion gemäß der Zusammensetzung von Tabelle 1 hergestellt.

**Tabelle 1: Beispielhafte Wasser-in-Öl-Emulsionszusammensetzung**

| **INCI-Bezeichnung** | **Gew.-%** |
|---|---|
| Carnitine | 5,00 |
| Isopropylpalmitate | 3,00 |
| Cera Microcristallina + Paraffinum Liquidum | 3,00 |
| Paraffinum Liquidum | 9,00 |
| C13-16 Isoparaffin | 6,00 |
| Polyglyceryl-3 Diisostearat | 1,43 |
| PEG-40 Sorbitan Perisostearate | 1,77 |
| Glycerin | 5,00 |
| Citric Acid | 0,09 |
| Sodium Citrate | 0,17 |
| Potassium Sorbate | 0,13 |
| Aqua | ad 100 |
| Magnesium Sulfate | 0,70 |
| | **100.00** |

Die Viskositätsmessungen wurden unter den folgenden Bedingungen vorgenommen:
**Gerätschaft:** Rheomat R 123 (einzige Drehzahl von 62,5 min⁻¹), Weithalsbecher, klares Glas, 150 ml (z.B. Rixius Nr. 110-150), Größe/Durchmesser: 5 cm; Spindel-Nr. 1.
**Messung:** Die Viskosität der Proben wird 24 h nach der Herstellung gemessen (Probentemperatur: 25°C+/-0,2°C). Vor der Messung der Viskosität einer Probe sollte dadurch eine Nulleinstellung erfolgen, dass man die Spindel anschließt und das Viskosimeter nullt. Sobald es genullt ist, sollte die Spindel blasenfrei bis zur Tauchmarke am Schaft in die Probe eingetaucht werden. Während einer Messzeit von 30 s können die Werte kontinuierlich abgelesen werden. Nach 30 s ist die Messung beendet.

Die erfindungsgemäße Zubereitung ist während der Herstellung und Lagerung stabil. D.h. es zeigten sich keinerlei Phasentrennung, Ausfällungen oder optische oder olfaktorische Veränderungen. Insbesondere blieb der eingekapselte hydrophile Wirkstoff Carnitin unverändert und verteilt in der Zubereitung.

Obwohl die Erfindung unter Bezugnahme auf bestimmte Ausführungsformen beschrieben worden ist, wird dem Fachmann klar sein, dass verschiedene Modifikationen, Veränderungen, Weglassungen und Austausche durchgeführt werden können, ohne vom Erfindungsgedanken abzuweichen. Die Erfindung sollte daher lediglich durch den Umfang der folgenden Ansprüche eingeschränkt sein. Die Merkmale von jedem abhängigen Anspruch können mit den Merkmalen von jedem der anderen abhängigen Ansprüche und jedem unabhängigen Anspruch kombiniert werden.

## Patentansprüche

1. Wasser-in-Öl-Emulsion umfassend ein Emulgatorsystem, das Sorbitanstearat oder ein Derivat davon und Polyglycerylstearat oder ein Derivat davon umfasst, und ein oder mehrere hydrophile Wirkstoffe, ausgenommen Dihydroxyaceton, **dadurch gekennzeichnet, dass** die Emulsion eine dynamische Viskosität im Bereich von 1000 bis 4000 mPa s bei 25°C aufweist.

2. Wasser-in-Öl-Emulsion nach Anspruch 1, die 1,0 bis 3,0 Gew.-% Sorbitanstearat oder ein Derivat davon in Bezug auf das Gesamtgewicht der Emulsion umfasst.

3. Wasser-in-Öl-Emulsion nach Anspruch 1 oder Anspruch 2, die 1,5 bis 2,0 Gew.-% Sorbitanstearat oder ein Derivat davon in Bezug auf das Gesamtgewicht der Emulsion umfasst.

4. Wasser-in-Öl-Emulsion nach einem der Ansprüche 1 bis 3, die 0,5 bis 2,0 Gew.-% Polyglycerylstearat oder ein Derivat davon in Bezug auf das Gesamtgewicht der Emulsion umfasst.

5. Wasser-in-Öl-Emulsion nach einem der Ansprüche 1 bis 4, die 1,0 bis 1,5 Gew.-% Polyglycerylstearat oder ein Derivat davon in Bezug auf das Gesamtgewicht der Emulsion umfasst.

6. Wasser-in-Öl-Emulsion nach einem der vorhergehenden Ansprüche, wobei das Sorbitanstearat Sorbitanperisostearat umfasst.

7. Wasser-in-Öl-Emulsion nach einem der vorhergehenden Ansprüche, wobei das Sorbitanstearat ein PEGyliertes Sorbitanperisostearat umfasst.

8. Wasser-in-Öl-Emulsion nach einem der vorhergehenden Ansprüche, wobei das Polyglycerylstearat Polyglyceryl-3 diisostearat umfasst.

9. Wasser-in-Öl-Emulsion nach einem der vorhergehenden Ansprüche, wobei das Emulgatorsystem nur aus einem Polyglycerylstearat und einem Sorbitanstearat besteht.

10. Wasser-in-Öl-Emulsion nach einem der vorhergehenden Ansprüche, wobei der Wirkstoff, den es einzukapseln gilt, in einer Menge von 1 bis 5 Gew.-% in Bezug auf das Gesamtgewicht der Emulsion einzukapseln ist.

11. Wasser-in-Öl-Emulsion nach Anspruch 10, wobei der hydrophile Wirkstoff, den es einzukapseln gilt, Carnitin umfasst.

12. Verfahren zur Herstellung der Wasser-in-Öl-Emulsion nach Anspruch 1, wobei man bei dem Verfahren Sorbitanstearat oder ein Derivat davon und Polyglycerylstearat oder ein Derivat davon mit einer Ölphase mischt und die Ölphase erhitzt, und die erhitzte Ölphase mit einer erhitzten wässrigen Phase unter Bildung der Wasser-in-ÖI-Emulsion homogenisiert.
